# EUROPÄISCHE PATENTANMELDUNG

(11) **EP 4 555 986 A1**
(43) Veröffentlichungstag der Anmeldung: **21.05.2025**
(21) Anmeldenummer: 24203331.4
(22) Anmeldetag: 27.09.2024
(51) Int. Cl.: A61F 15/00, B65D 25/22, B65D 83/02, B65D 83/08

(54) **SPENDEREINRICHTUNG FÜR SLIPEINLAGEN UND TAMPONS**

(30) Priorität: 17.11.2023 DE 202023106786 U
(71) Anmelder: TEMCA GmbH & Co. KG, 07554 Pölzig (DE)
(72) Erfinder: BURGHARDT, Axel, 07549 Gera (DE)
(74) Vertreter: Liebl, Thomas

(57) **Zusammenfassung**

Die Erfindung betrifft eine Spendereinrichtung für Slipeinlagen und Tampons mit einem Gehäuse (2), das ausgebildet ist, in dem Gehäuseinnenraum (17) mehrere Slipeinlagen und mehrere Tampons getrennt voneinander aufzunehmen, wobei das Gehäuse (2) eine Slipeinlagen-Entnahmeöffnung (9), über die die Slipeinlagen entnehmbar sind, und eine Tampon-Entnahmeöffnung (10), über die die Tampons entnehmbar sind, aufweist. Das Gehäuse (2) ist kastenförmig ausgebildet und weist einen Spenderkorpus (3) mit einer Rückwand (4) und einer sich nach vorne, von der Rückwand (4) weg erstreckenden Korpus-Wandanordnung (5) auf, wobei der Spenderkorpus (3) einen sich an die Rückwand (4) nach vorne hin anschließenden Aufnahmeraum (6) aufweist, der wenigstens einen Bestandteil des Gehäuseinnenraums (17) ausbildet und der von der Korpus-Wandanordnung (5) wenigstens bereichsweise umschlossen ist, wobei der spenderkorpusseitige Aufnahmeraum (6) nach vorne hin, in Richtung von der Rückwand (4) weg, offen ist und mittels eines, eine Vorderwand (7) des Gehäuses (2) ausbildenden Spenderdeckels (8) als weiterem Gehäuseteil lösbar verschließbar ist. Die Slipeinlagen-Entnahmeöffnung (9) und die Tampon-Entnahmeöffnung (10) sind in der Vorderwand (7) ausgebildet.

## Beschreibung

Die Erfindung betrifft eine Spendereinrichtung für Slipeinlagen und Tampons nach dem Oberbegriff des Anspruches 1.

Es gibt verschiedene Damenhygieneprodukte für die Menstruation und den Alltag, wie beispielsweise Binden, Slipeinlagen und Tampons als gängigste Hygieneprodukte. Während Binden und Slipeinlagen in der Regel eine flache, plattenartige Form aufweisen, die gut stapelbar ist, handelt es sich bei einem Tampon in der Regel um einen gepressten Watte- oder Mullbausch, der eine längliche zylindrische Form aufweist. Üblicherweise werden Binden und Slipeinlagen, die nachfolgend oberbegriffsartig stets als Slipeinlagen bezeichnet werden, ebenso wie Tampons in Verpackungen aus Pappe verkauft, die bei einer privaten Nutzung beispielsweise in Badezimmerschränken, Toilettenschränken oder dergleichen aufbewahrt werden, wobei die Slipeinlagen beziehungsweise Tampons dann von dort bedarfsweise entnommen werden.

Für viele kommt die Periode oft unerwartet und unregelmäßig, wodurch die Gefahr besteht, dass die benötigten Periodenprodukte nicht sofort zur Hand sind. Aus diesem Grund, und insbesondere auch zur Enttabuisierung des Themas "Menstruation", gibt es mittlerweile verstärkte Bestrebungen in Schulen, an Universitäten, am Arbeitsplatz und in öffentlichen Einrichtungen sogenannte Tampon- und Slipeinlagenspender zur Verfügung zu stellen, so wie dies beispielsweise bei Toilettenpapier und Seife ganz selbstverständlich der Fall ist.

Eine derartige Spendereinrichtung für Slipeinlagen und Tampons ist aus der DE 201 04 616 U1 bekannt, bei dem ein Gehäuse vorgesehen ist, in dem die Slipeinlagen und die Tampons derart angeordnet sind, dass sie aufeinander folgend einer Ausgabeöffnung zur Entnahme zuführbar sind. Das Gehäuse weist an seinem oberen Ende einen Deckelteil auf, der abgenommen werden kann, damit das Gehäuse von oben her mit Slipeinlagen befüllt werden kann bzw. die Slipeinlagen dort zusammen mit ihrer handelsüblichen Verpackung eingesetzt werden können. Ein unterer Eckbereich des Gehäuses ist ausgespart, so dass die jeweils unterste Slipeinlage des Stapels mit einem Endbereich in der Entnahmeöffnung freiliegt und mit ihrem restlichen Bereich auf einer Bodenwand aufliegt, so dass der in der Entnahmeöffnung befindliche Endbereich zur Entnahme leicht manuell ergriffen werden kann. Zudem können an der Vorderwand des Gehäuses Tamponspender angebracht werden, die jeweils ein nach oben offenes oder durch einen Deckel verschließbares Gehäuseteil aufweisen, in dem die Tampons übereinander stapelförmig angeordnet werden. Auch hier ist im Bereich des unteren Endes des Gehäuseteils eine Entnahmeöffnung vorgesehen, durch die der jeweils unterste Tampon manuell entnommen bzw. herausgezogen werden kann. Das Gehäuseteil wird an der Vorderwand des Gehäuses für die Slipeinlagen bevorzugt durch Kleben angebracht. Für eine Wandmontage des Gehäuses für die Slipeinlagen sind zum Beispiel Klebeflächen auf der Rückseite des Gehäuses angeordnet.

Eine derartige Spendereinrichtung ist für einen funktionssicheren Betrieb in Schulen, Universitäten, am Arbeitsplatz und im öffentlichen Bereich nur sehr wenig praktikabel, insbesondere im Hinblick auf die kombinierte Anordnung zur Ausbildung einer Spendereinrichtung für Slipeinlagen und Tampons. So können beispielsweise durch die vorgesehenen Klebeflächen die einzelnen Gehäuseteile leicht voneinander entfernt bzw. manipuliert werden.

Demgegenüber ist es Aufgabe der vorliegenden Erfindung, eine technisch hochwertige Spendereinrichtung für Slipeinlagen und Tampons zu schaffen, die bei hoher Funktionssicherheit kompakt aufgebaut ist und eine zuverlässige Entnahme von sowohl Slipeinlagen und Tampons ermöglicht.

Diese Aufgabe wird gelöst mit den Merkmalen des Anspruches 1. Vorteilhafte Ausgestaltungen sind Gegenstand der darauf rückbezogenen Unteransprüche.

Gemäß Anspruch 1 ist eine Spendereinrichtung für Slipeinlagen und Tampons vorgesehen, die durch ein Gehäuse gekennzeichnet ist, das ausgebildet ist, in dem Gehäuseinnenraum mehrere Slipeinlagen und mehrere Tampons getrennt voneinander aufzunehmen. Das Gehäuse weist eine Slipeinlagen-Entnahmeöffnung, über die die Slipeinlagen entnehmbar sind, und eine Tampon-Entnahmeöffnung, über die die Tampons entnehmbar sind, auf. Das Gehäuse ist kastenförmig, insbesondere rechteck- und kastenförmig, ausgebildet und weist einen Spenderkorpus mit einer Rückwand und einer sich nach vorne, von der Rückwand weg erstreckenden Korpus-Wandanordnung auf. Der Spenderkorpus weist weiter einen sich an die Rückwand nach vorne hin anschließenden Aufnahmeraum auf, der wenigstens einen Bestandteil des Gehäuseinnenraums ausbildet und der von der Korpus-Wandanordnung wenigstens bereichsweise umschlossen ist, wobei der spenderkorpusseitige Aufnahmeraum nach vorne hin, in Richtung von der Rückwand weg, offen ist und mittels eines, eine Vorderwand des Gehäuses ausbildenden Spenderdeckels als einem weiteren Gehäuseteil lösbar verschließbar ist. Die Slipeinlagen-Entnahmeöffnung und die Tampons-Entnahmeöffnung sind in der Vorderwand ausgebildet.

Mit einer derartigen Ausgestaltung einer erfindungsgemäßen Spendereinrichtung für sowohl Slipeinlagen als auch Tampons gelingt es, eine robuste, technisch hochwertige und einfach herzustellende Vorrichtung bereitzustellen, die zum einen eine bequeme Entnahme der Slipeinlagen und/oder Tampons von der Vorderwand des Gehäuses ermöglicht und zudem auch das Befüllen selbst wesentlich vereinfacht, weil hierzu einfach nur noch der die Vorderwand ausbildende Spenderdeckel abgenommen werden muss, so dass ein bequemes Befüllen der Spendereinrichtung von der gut zugänglichen Vorderseite her möglich ist. Dies gelingt insbesondere mit einem Spenderdeckel der schwenkbar am Schwenkerkorpus angelenkt ist, insbesondere in einem, bezogen auf die Hochachsenrichtung, unteren Endbereich des Spenderkorpus schwenkbar angelenkt ist, da damit im geöffneten, aufgeschwenkten Zustand des Spenderdeckels der spenderkorpusseitige Aufnahmebereich und damit der Gehäuseinnenraum für ein Befüllen der Spendereinrichtung sehr gut von der Vorderseite aus zugänglich ist. Zudem ist der Spenderdeckel durch die schwenkbare Anlenkung auch im geöffneten Zustand unverlierbar am Spenderkorpus gehaltert. Grundsätzlich besteht aber natürlich auch die Möglichkeit, den Spenderdeckel als Ganzes vom Spenderkorpus abzunehmen, wenn dies für bestimmte Anwendungsfälle explizit gewünscht sein sollte.

Gemäß einer besonders bevorzugten konkreten Ausgestaltung weist die Korpus-Wandanordnung zwei auf gegenüberliegenden Seiten des Aufnahmeraums liegende und sich in Hochachsenrichtung erstreckende Korpus-Seitenwände auf, an denen der Spenderdeckel schwenkbar angelenkt ist. Auch hier ist es dann bevorzugt, wenn der Spenderdeckel jeweils an einem, bezogen auf die Hochachsenrichtung, unteren Endbereich der Korpus-Seitenwände schwenkbar angelenkt ist, so dass der Spenderdeckel nach unten aufschwenkbar ist. Eine derartige schwenkbare Anlenkung an den Korpus-Seitenwänden lässt sich auf besonders einfache Weise herstellen und führt zu einer funktionssicheren schwenkbaren Anlenkung des Spenderdeckels.

Gemäß einer weiteren besonders bevorzugten Ausgestaltung ist vorgesehen, dass sich, bezogen auf den geschlossenen Zustand des Spenderdeckels, an den die Vorderwand ausbildenden Bereich des Spenderdeckels nach hinten hin, also in Richtung zur Rückwand hin, eine Deckel-Wandanordnung anschließt. Eine derartige konkrete Ausgestaltung ermöglicht es, den Spenderdeckel bevorzugt so auszubilden, dass er ebenfalls einen sich an die Vorderwand nach hinten in Richtung Rückwand anschließenden deckelseitigen Aufnahmeraum aufweist, der von der Deckel-Wandanordnung wenigstens bereichsweise umschlossen ist. Dadurch kann der deckelseitige Aufnahmeraum zusammen mit dem korpusseitigen Aufnahmeraum den Gehäuseinnenraum ausbilden. Bei einer derartigen konkreten Ausgestaltung, bei der der Gehäuseinnenraum zum einen deckelseitig und zum anderen korpusseitig ausgebildet wird, ergibt sich zum Beispiel der Vorteil, dass bei geöffneten Spenderdeckel die im Gehäuseinnenraum korpusseitig angeordneten bzw. aufgenommen Slipeinlagen und Tampons besonders gut zugänglich sind. Zudem bewirkt die Deckel-Wandanordnung eine relativ stabile Ausgestaltung des Deckels als solches, der somit klapperfrei und damit technisch hochwertig an dem Spenderkorpus angeordnet werden kann.

Gemäß einer weiteren besonders bevorzugten Ausgestaltung weist die Deckel-Wandanordnung zwei den Korpus-Seitenwänden zugeordnete Deckel-Seitenwände auf, wobei die zwei Korpus-Seitenwände in einem, dem Spenderdeckel zugewandten vorderen freien Endbereich nach innen versetzt, insbesondere abgekröpft und/oder verjüngt, sind, so dass die Korpus-Seitenwände jeweils einen hinteren Korpus-Seitenwandabschnitt und einen sich daran nach vorne, in Richtung zum Spenderdeckel hin anschließenden, gegenüber dem hinteren Korpus-Seitenwandabschnitt nach innen, in Richtung Aufnahmeraum versetzten, vorderen Korpus-Seitenwandabschnitt aufweisen. Die Deckel-Seitenwände umgreifen dann, bezogen auf den geschlossenen Zustand des Spenderdeckels, den nach innen versetzten vorderen Korpus-Seitenwandabschnitt von außen her, vorzugsweise mit einem Spaltabstand und sind im Wesentlichen in einer Flucht mit den jeweils zugeordneten hinteren Korpus-Seitenwandabschnitten ausgerichtet. Besonders bevorzugt ist bei dieser Ausführungsform vorgesehen, dass die Deckel-Seitenwände und die jeweils zugeordneten hinteren Korpus-Seitenwandabschnitte im geschlossenen Zustand des Spenderdeckels und damit des Gehäuses im Wesentlichen unmittelbar aneinander angrenzen und eine nach außen sichtbare gemeinsame Gehäuse-Seitenwand ausbilden. Mit einer derart konkreten Ausgestaltung gelingt es, ein besonders funktionssicheres und technisch hochwertiges klemmfreies Schließen des Spenderdeckels zu bewirken, da die nach innen versetzten vorderen Korpus-Seitenwandabschnitte nicht mit dem Deckel-Seitenwänden kollidieren können, sondern beim Schließen des Spenderdeckels einfach an diesen vorbei in den deckelseitigen Aufnahmeraum eingreifen können. Diese vorderen Korpus-Seitenwandabschnitte bewirken somit ein hochwertiges, funktionssicheres sowie geführtes Schließen des Spenderdeckels. Zudem ergibt sich durch die im Wesentlichen fluchtende Anordnung der hinteren Korpus-Seitenwandabschnitte mit den Deckel-Seitenwänden ein ästhetisch hochwertiger Gesamteindruck, da der Eindruck einer im Wesentlichen durchgehenden gemeinsamen Gehäuse-Seitenwand vermittelt wird, und zwar ohne störende, sichtbare Kanten und Vorsprünge, die nicht nur unansehnlich sind, sondern zudem auch eine gewissen Verletzungsgefahr beinhalten und auch stark zum Verschmutzen durch Schmutzanlagerungen, insbesondere in feuchter Umgebung, neigen.

In Verbindung mit einem derartigen konkreten Aufbau ist es weiter besonders bevorzugt, wenn der Spenderdeckel mit seinen Deckel-Seitenwänden schwenkbar im Bereich der vorderen Korpus-Seitenwandabschnitte angelenkt ist. Die Anlenkung ist hierbei bevorzugt so durchgeführt und/oder die Deckel-Seitenwände sind bevorzugt so ausgebildet, dass der Spenderdeckel im aufgeschwenkten und damit geöffneten Zustand, insbesondere in einem um 90 Grad von dem Spenderkorpus weg aufgeschwenkten und geöffneten Zustand, mit seinem schwenkachsenseitigen unteren Deckel-Wandkantenbereich von der Rückwand beabstandet ist. Dies hat den Vorteil, dass damit zuverlässig vermieden werden kann, dass zum Beispiel bei einer Wandbefestigung der Spendereinrichtung eine Deckelkante in Kontakt mit den Befestigungswandbereich gelangt bzw. dort einschneidet, was zu einer Beschädigung von sowohl dem Spenderdeckel als auch der Befestigungswand führen kann.

Die Korpus-Wandanordnung kann des Weiteren, bezogen auf einen in Hochachsenrichtung oberen Bereich des Gehäuses, eine sich wenigstens zwischen den hinteren Korpus-Seitenwandabschnitten erstreckende Korpus-Oberwand aufweisen, an die im geschlossenen Zustand des Spenderdeckels eine, bezogen auf einen in Hochachsenrichtung oberen Bereich des Gehäuses, sich zwischen den Deckel-Seitenwänden erstreckende Deckel-Oberwand im Wesentlichen unmittelbar angrenzt und/oder anliegt. Dabei ist bevorzugt vorgesehen, dass die Deckel-Oberwand und die Korpus-Oberwand im Wesentlichen in einer Flucht liegen und im geschlossenen Zustand des Spenderdeckels und damit des Gehäuses eine gemeinsame Gehäuse-Oberwand bilden. Hier gilt bezüglich der sich dadurch ergebenden Vorteile wiederum das zuvor zur Gehäuse-Seitenwand gesagte in analoger Weise, nämlich dass dadurch nicht nur ein hochwertiger ästhetischer Gesamteindruck erreicht wird, sondern darüber hinaus auch unerwünschte Vorsprünge und Kanten vermieden werden können. Zudem führt eine derartige zusätzliche Oberwand sowohl im Bereich des Spenderkorpus als auch im Bereich des Spenderdeckels zu einer weiteren Stabilisierung von sowohl dem Spenderkorpus als auch dem Spenderdeckel, so dass ein insgesamt stabiler Gehäuseaufbau zur Verfügung gestellt wird, mithin das Gehäuse insgesamt klapperfrei und robust ausgebildet ist.

Insbesondere um ein Verkannten und kollisionsfreies Einführen des vorderen Korpus-Seitenwandabschnitte in den deckelseitigen Aufnahmeraum zu erreichen, wie dies beim Schließen des Spenderdeckels der Fall ist, ist gemäß einer besonders bevorzugten Ausgestaltung vorgesehen, dass, jeweils bezogen auf die Hochachsenrichtung, zumindest ein vorderer Endkantenbereich einer Oberkante der vorderen Korpus-Seitenwandabschnitte tiefer liegt als die Deckel-Oberwand. Um dies zu erreichen ist bevorzugt vorgesehen, dass der vordere Entkantenbereich einer Oberkante der vorderen Korpus-Seitenwandabschnitte beispielsweise angefast oder angeschrägt ist.

Weiter ist gemäß einer besonders bevorzugten Ausgestaltung vorgesehen, dass ein Betätigungselement einer Schließvorrichtung, vorzugsweise ein Drehbetätigungselement einer Drehschließvorrichtung, in der Deckel-Oberwand oder in der Korpus-Oberwand im Wesentlichen oberflächenbündig und von außen her zugänglich einliegt, und bei dessen Betätigung dann ein im Gehäuseinnenraum an einer der Oberwände angeordnetes Schließelement, vorzugsweise ein Drehschließelement, zum Beispiel ein Haken oder dergleichen, in Eingriff und außer Eingriff mit einem im Gehäuseinnenraum an der anderen Oberwand angeordneten Schließgegenelement bringbar ist. Durch die einliegende Anordnung des Betätigungselements gibt es keine störenden vorspringenden Kantenbereiche. Zudem kann eine derartige Betätigung des Betätigungselements einfach von außen, zum Beispiel mittels eines Schlüssels, durchgeführt werden. Mittels der Schließvorrichtung wird zudem ein funktionssicheres Halten des Spenderdeckels im geschlossenen Zustand erzielt. Grundsätzlich gibt es selbstverständlich auch andere Möglichkeiten, den Spenderdeckel in dem geschlossenen Zustand zu haltern bzw. zu verriegeln, beispielsweise eine Klemm- und/oder Rastverbindung, um nur ein Beispiel zu nennen.

Gemäß einer weiteren besonders bevorzugten erfindungsgemäßen Ausführungsform ist im Gehäuseinnenraum ein Gehäuseeinsatz angeordnet, der eine sich in Hochachsenrichtung erstreckende Slipeinlagen-Aufnahmekammer und eine davon getrennte, sich ebenfalls in Hochachsenrichtung erstreckende Tampon-Aufnahmekammer ausbildet. Weiter münden die Tampon-Aufnahmekammer in der Tampon-Entnahmeöffnung und die Slipeinlagen-Aufnahmekammer in der Slipeinlagen-Entnahmeöffnung. Ein derartiger Gehäuseeinsatz wird beispielsweise durch ein separates Bauteil gebildet, das im Gehäuseinnenraum zu montieren ist. Dadurch ergeben sich dann, zum Beispiel in Verbindung mit unterschiedlichen Gehäuseeinsätzen, flexible Anwendungsmöglichkeiten, zum Beispiel im Hinblick auf die Anpassung der Größe der Slipeinlagen-Aufnahmekammer bzw. der Tampon-Aufnahmekammer. Der Gehäuseeinsatz kann grundsätzlich aber auch integraler Bestandteil der Spendereinrichtung sein, so zum Beispiel integraler Bestandteil des Spenderkorpus bzw. integraler Bestandteil des Spenderdeckels sein. Auf jeden Fall bewirkt ein derartiger Gehäuseeinsatz eine einfache und funktionssichere Trennung des Gehäuseinnenraums in die beiden gewünschten Aufnahmekammern für die Slipeinlagen und die Tampons.

Für einen kompakten Aufbau ist es zudem besonders vorteilhaft, wenn die Tampon-Aufnahmekammer und die Slipeinlagen-Aufnahmekammer in Querrichtung gesehen nebeneinanderliegen. Insbesondere in Verbindung mit einem derartigen nebeneinanderliegenden Aufbau der beiden Aufnahmekammern, aber auch unabhängig davon, ist es vorteilhaft, wenn die Tampon-Entnahmeöffnung und die Slipeinlagen-Entnahmeöffnung in Querrichtung gesehen nebeneinander in einem, bezogen auf die Hochachsenrichtung, unteren Vorderwandbereich angeordnet sind.

Des Weiteren ist es für einen kompakten Aufbau der Spendereinrichtung vorteilhaft, wenn sich der Gehäuseeinsatz gemäß einer besonders bevorzugten konkreten Ausgestaltung zwischen der Rückwand und der Vorderwand erstreckt, insbesondere im Wesentlichen unmittelbar an die Vorderwand und an die Rückwand angrenzt.

Insbesondere für den Fall eines durch ein separates Bauteil gebildeten Gehäuseeinsatzes ist es vorteilhaft, wenn der Gehäuseeinsatz mit in Querrichtung gegenüberliegenden Gehäuseeinsatz-Seitenbereichen an der Korpus-Wandanordnung, vorzugsweise an den Korpus-Seitenwänden der Korpus-Wandanordnung, gehaltert ist. Ganz allgemein und unabhängig von den Gehäuseeinsatz-Seitenbereichen gesehen, kann diese Halterung lösbar ausgebildet sein, so dass der Gehäuseeinsatz beispielsweise mittels einer Klemm- und/oder Schraub- und/oder Rastverbindung lösbar an dem Spenderkorpus, vorzugsweise an den Korpus-Seitenwänden der Korpus-Wandanordnung, gehaltert ist. Besonders bevorzugt ist jedoch eine stabile und feste Verbindung des Gehäuseeinsatzes mit dem Spenderkorpus, vorzugsweise wenigstens mit den Korpus-Seitenwänden der Korpus-Wandanordnung, zum Beispiel durch eine nur durch Zerstörung, das heißt bedingt lösbare Verbindung, wie zum Beispiel wenigstens eine Nietverbindung und/oder wenigstens eine Klebeverbindung.

Der Gehäuseeinsatz kann zudem gemäß einer weiteren besonders bevorzugten Ausführungsform in einem, bezogen auf die Hochachsenrichtung, unteren Bereich der Slipeinlagen-Aufnahmekammer eine, vorzugsweise im Wesentlichen horizontal ausgerichtete und/oder plattenförmige, Gehäuseeinsatz-Bodenwand aufweisen, die eine Auflagefläche für wenigstens eine darauf aufliegende und in der Slipeinlagen-Aufnahmekammer aufgenommene Slipeinlage ausbildet, vorzugsweise eine Auflagefläche für eine unterste Slipeinlage eines Stapels aus mehreren, in der Slipeinlagen-Aufnahmekammer aufgenommenen Slipeinlagen ausbildet. Mit einer derartigen Gehäuseeinsatz-Bodenwand wird somit eine einfache und funktionssichere Auflagefläche für die Slipeinlagen zur Verfügung gestellt. In Verbindung mit der Slipeinlagen-Aufnahmekammer ist es von besonderem Vorteil, wenn die Slipeinlagen-Aufnahmekammer bei geschlossenem Spenderdeckel von der Rückwand, der Vorderwand, einer Gehäuse-Seitenwand und einer Wand der Tampon-Aufnahmekammer begrenzt ist und bei geöffnetem Spenderdeckel zumindest nicht mehr durch die Vorderwand begrenzt ist und damit für eine Beschickung mit Slipeinlagen frei zugänglich ist. Dies führt zu einem insgesamt wenig bauteilintensiven, herstellungstechnisch einfach herstellbaren Aufbau einer Slipeinlagen-Aufnahmekammer.

Weiter ist es besonders bevorzugt, wenn die Gehäuseeinsatz-Bodenwand die Slipeinlagen-Entnahmeöffnung, die als unterkantenseitige Aussparung in der Vorderwand ausgebildet ist, nach unten hin begrenzt In diesem Zusammenhang ist es besonders vorteilhaft, wenn die Gehäuseeinsatz-Bodenwand gleichzeitig auch das Gehäuse nach unten hin begrenzt und/oder einen Teil der Gehäuse-Bodenwand ausbildet und/oder sich zwischen der Vorderwand und der Rückwand erstreckt, insbesondere im Wesentlichen unmittelbar an die Vorderwand und/oder an die Rückwand angrenzt. Damit wird eine vorteilhafte Materialdopplung im Bereich der Gehäuse-Bodenwand vermieden und bildet somit die Gehäuseeinsatz-Bodenwand in einer vorteilhaften Doppelfunktion gleichzeitig auch einen Bestandteil der Gehäuse-Bodenwand. Dadurch lässt sich der Bauteilaufwand bei der Herstellung der Spendereinrichtung deutlich reduzieren.

Die Gehäuseeinsatz-Bodenwand weist an ihrem der Slipeinlagen-Entnahmeöffnung zugewandten Vorderkantenbereich, vorzugsweise in etwa mittig mit Bezug auf die Quererstreckung der Slipeinlagen-Entnahmeöffnung in der Vorderwand, eine Ausnehmung, vorzugsweise eine halbkreisförmige Ausnehmung, als Fingereingriffsöffnung auf. Damit können die Slipeinlagen sehr einfach und ungehindert manuell ergriffen werden.

Des Weiteren kann gemäß einer besonders bevorzugten Ausführungsform vorgesehen sein, dass Gehäuseeinsatz-Bodenwand mit einem Verbindungsbereich an eine der Korpus-Seitenwände angrenzt und mit der Korpus-Seitenwand verbunden ist. Dadurch lässt sich eine stabile Anordnung der Gehäuseeinsatz-Bodenwand erzielen. Weiter kann bevorzugt vorgesehen sein, dass der Verbindungsbereich durch eine in Hochachsenrichtung nach oben gerichtete Umbiegung der Gehäuseeinsatz-Bodenwand gebildet ist, die im montierten Zustand flächig an der zugeordneten Korpus-Seitenwand anliegt. Mit einer derartigen flächigen Anlage wird eine besonders stabile Anbindung der Gehäuseeinsatz-Bodenwand an die zugeordnete Korpus-Seitenwand erzielt und damit auch insgesamt äußerst kompakter und stabiler Aufbau des Gehäuses der Spendereinrichtung.

Zudem ist gemäß einer besonders bevorzugten Ausgestaltung vorgesehen, dass die Korpus-Wandanordnung, bezogen auf einen in Hochachsenrichtung unteren Bereich des Gehäuses, eine Korpus-Bodenwand aufweist, die sich wenigstens im Bereich unterhalb der Tampon-Aufnahmekammer erstreckt, bevorzugt sich wenigstens im Bereich unterhalb der Tampon-Aufnahmekammer zwischen der Vorderwand und der Rückwand erstreckt, höchst bevorzugt im Wesentlichen unmittelbar an die Vorderwand und an die Rückwand angrenzt. Mit einem derartigen Aufbau der Spendereinrichtung und insbesondere des Spenderkorpus mit einer Korpus-Bodenwand wird zu einem eine vorteilhafte Stabilisierung des Spenderkorpus erreicht und zum andere auch auf einfache Weise eine Auflagefläche für Tampons ausbildbar, was die nachfolgenden Ausführungen zu den bevorzugten Ausgestaltungen hierzu zeigen:
So kann beispielsweise gemäß einer besonders bevorzugten konkreten Ausgestaltung die Korpus-Bodenwand im Bereich der Gehäuseeinsatz-Bodenwand ausgespart sein und sich unmittelbar an die Gehäuseeinsatz-Bodenwand anschließen. Dadurch kann entsprechend einer weiteren besonders bevorzugten Ausgestaltung vorgesehen werden, dass die Gehäuseeinsatz-Bodenwand und die Korpus-Bodenwand im Wesentlichen in einer Flucht liegen und im geschlossenen Zustand des Spenderdeckels und damit des Gehäuses eine gemeinsame Gehäuse-Bodenwand bilden.

Wie bereits zuvor ausgeführt ist es dann vorteilhaft möglich, dass die Korpus-Bodenwand eine Auflagefläche für Tampons ausbildet. Alternativ oder zusätzlich kann gemäß einer weiteren besonders bevorzugten Ausgestaltung vorgesehen werden, dass ein der Vorderwand zugewandter Vorderkantenbereich an den Unterkantenbereich der in der Vorderwand ausgebildeten Tampon-Entnahmeöffnung angrenzt. Damit ist ein einfacher Zugriff auf die auf der Auflagefläche aufliegenden Tampons durch die Tampon-Entnahmeöffnung möglich.

In diesem Zusammenhang ist es weiter vorteilhaft, wenn die Korpus-Bodenwand und damit die Auflagefläche der Korpus-Bodenwand, bezogen auf die Hochachsenrichtung, tiefer liegt als eine Unterkante der Tampon-Entnahmeöffnung. Damit wird auf einfache Weise sichergestellt, dass die eine zylindrische Form aufweisenden Tampons nicht in unerwünschter Wiese aus der Tampon-Entnahmeöffnung fallen und somit funktionssicher und zuverlässig auf der Auflagefläche der Korpus-Bodenwand aufliegen und gehaltert werden.

Gemäß einer weiteren besonders bevorzugten Ausführungsform kann der der Vorderwand zugewandte Vorderkantenbereich der Korpus-Bodenwand eine, bezogen auf die Hochachsenrichtung, konvexe, vorzugsweise oberseitig abgerundete, Aufwölbung aufweisen. Mit einer derartigen Aufwölbung wird auf einfache Weise sichergestellt, dass keine scharfen Kanten im Bereich der Tampon-Entnahmeöffnung vorhanden sind, die beim manuellen Eingriff in die Tampon-Entnahmeöffnung zur Entnahmeentnahme gegebenenfalls zu einer Verletzung der Finger führen könnten. Weiter ist es besonders vorteilhaft, wenn von der konvexen Aufwölbung ausgehend ein Anlageschenkel im Wesentlichen geradlinig nach unten abragt, wobei der Anlageschenkel bei geschlossenem Spenderdeckel, bevorzugt ohne Spaltabstand und/oder in einer flächigen Anlage, an die Vorderwand angrenzt. Damit wird eine funktionssichere Anlage und Abstützung der Korpus-Bodenwand bei geschlossenem Spenderdeckel sichergestellt.

Gemäß einer weiteren besonders bevorzugten Ausgestaltung kann die Tampon-Aufnahmekammer, bezogen auf die Hochachsenrichtung, im Bereich oberhalb der Korpus-Bodenwand eine Einfüllkammer aufweisen, deren Einfüllkammer-Bodenwand beabstandet oberhalb der Korpus-Bodenwand liegt. Diese Einfüllkammer-Bodenwand ist bevorzugt als Schrägwand ausgebildet, die von einem der Vorderwand zugewandten ersten Schrägwandbereich ausgehend in Richtung zur Rückwand hin schräg abfällt, und zwar dergestalt, dass ein dem ersten Schrägwandbereich gegenüberliegender, der Rückwand zugewandter zweiter Schrägwandbereich, vorzugsweise dessen endseitige Schrägwandkante, einen Spaltabstand zur Rückwand aufweist, wobei der Spaltabstand zur Rückwand so bemessen ist, dass über ihn Tampons von der Einfüllkammer in den Bereich der Korpus-Bodenwand gelangen können. Mit einer derartigen Schrägwand wird auf einfache Weise sichergestellt, dass die über die Einfüllkammer eingefüllten Tampons kontrolliert über einen hinteren, von der Tampon-Entnahmeöffnung abgewandten Bereich in den Bereich der Korpus-Bodenwand gelangen können, was wesentlich dazu beiträgt, dass die Tampons nicht aus der Tampon-Entnahmeöffnung fallen können. Denn sobald sich in diesem hinteren Bereich der nach hinten abfallenden Schrägwand zu viele Tampons befinden, wird der Nachfluss der Tampons selbsttätig unterbunden und erst dann wieder freigegeben, wenn eine entsprechende Anzahl von Tampons aus dem Bereich der Korpus-Bodenwand entnommen worden ist. Durch die Schrägwand wird zudem sichergestellt, dass es zu keinen unerwünschten Verstopfungen kommen kann.

Der Spaltabstand kann hier zum Beispiel durch einen sich in Querrichtung erstreckenden Längsschlitz gebildet sein, so dass die Tampon nur entsprechend quer ausgerichtet durch den Längsschlitz nach unten auf die Korpus-Bodenwand fallen können, wobei der Längsschlitz selbstverständlich eine Größe aufweist, die um ein definiertes Maß größer ist als der größte in der Einfüllkammer aufnehmbare Tampon. Damit wird die Funktionssicherheit der Spendereinrichtung im Bereich der Tampon-Aufnahmekammer auf einfache Weise realisiert.

Die Einfüllkammer ist gemäß einer weiteren besonders bevorzugten Ausgestaltung in Umfangsrichtung gesehen wenigstens bereichsweise durch eine Einfüllkammer-Wandanordnung, bevorzugt durch eine Einfüllkammer-Wandanordnung und die Rückwand, höchst bevorzugt durch eine Einfüllkammer-Wandanordnung, die Rückwand und eine Korpus-Seitenwand, begrenzt und weist an ihrer, bezogen auf die Hochachsenrichtung, Oberseite eine obere Einfüllöffnung auf, wobei vorzugsweise vorgesehen ist, dass die obere Einfüllöffnung ganz einfach durch eine oben offen ausgebildete Einfüllkammer ausgebildet wird. Mit einem derartigen Aufbau ist sichergestellt, dass die Tampons in der Einfüllkammer aufgenommen werden und diese Einfüllkammer nur über die Einfüllöffnung zugänglich ist. Dementsprechend ist im geöffneten Zustand des Spenderdeckels im Wesentlichen die Einfüllöffnung für eine Befüllung mit Tampons zugänglich.

Weiter ist es bevorzugt, wenn sich die Einfüllkammer, bezogen auf die Hochachsenrichtung, so weit nach oben erstreckt, dass die Einfüllöffnung, vorzugsweise mit einem Spaltabstand, an eine Korpus-Oberwand der Korpus-Wandanordnung angrenzt. Damit ergibt sich ein großes Füllvolumen für die Einfüllkammer.

Die Einfüllkammer-Wandanordnung kann gemäß einer weiteren besonders bevorzugten Ausgestaltung eine bei geschlossenem Spenderdeckel der Vorderwand zugeordnete Einfüllkammer-Vorderwand aufweisen, die bei geöffnetem Spenderdeckel von außen sichtbar ist. Mittels dieser Einfüllkammer-Vorderwand ist sichergestellt, dass die Tampons beim Öffnen des Spenderdeckels nicht unkontrolliert aus der Spendereinrichtung fallen. Gemäß einer besonders bevorzugten Ausgestaltung hierzu ist vorgesehen, dass die Einfüllkammer-Vorderwand bei geschlossenem Spenderdeckel im Wesentlichen parallel zur Vorderwand ausgerichtet ist und/oder an die Vorderwand angrenzt, höchst bevorzugt flächig an der Vorderwand anliegt. Dadurch wird sichergestellt, dass ein insgesamt kompakter Gehäuseaufbau realisierbar ist.

Die Einfüllkammer-Vorderwand kann gemäß einer weiteren besonders bevorzugten Ausgestaltung im Bereich ihrer, bezogen auf die Hochachsenrichtung, Oberkante eine Ausnehmung, vorzugsweise eine halbkreisförmige Ausnehmung, als eine die obere Einfüllöffnung erweiternde seitliche Einfüllöffnung aufweisen. Damit wird das Einfüllen der Tampons in die Einfüllkammer erleichtert, und zwar insbesondere für den Fall, dass die obere Einfüllöffnung bei geöffnetem Spenderdeckel teilweise von einer Korpus-Oberwand der Korpus-Wandanordnung überdeckt ist.

Die Einfüllkammer-Wandanordnung kann gemäß einer weiteren besonders bevorzugten Ausgestaltung zudem eine Einfüllkammer-Seitenwand aufweisen, die sich zur Abtrennung der Tampon-Aufnahmekammer von der Slipeinlagen-Aufnahmekammer im Gehäuseinnenraum, vorzugsweise in einem mittleren Bereich des Gehäuseinnenraums, in Richtung zur Rückwand hin winklig, vorzugsweise in etwa mit einem Winkel von 90 Grad, an die Einfüllkammer-Vorderwand anschließt und sich bis zur Rückwand erstreckt, bevorzugt an die Rückwand angrenzt, höchst bevorzugt an der Rückwand anliegt. Damit dient die Einfühlkammer-Seitenwand in einer vorteilhaften Doppelfunktion gleichzeitig zur funktionssicheren Abtrennung der Tampon-Aufnahmekammer von der Slipeinlagen-Aufnahmekammer.

Weiter ist es gemäß eines besonders bevorzugten Ausführungsform vorteilhaft, wenn die Gehäuseeinsatz-Bodenwand, vorzugsweise in einem bezogen auf die Hochachsenrichtung unteren Bereich der Einfüllkammer-Seitenwand, von der Einfüllkammer-Seitenwand abragt, vorzugsweise im Wesentlichen horizontal abragt, und/oder an der Einfüllkammer-Seitenwand angebunden ist. Auch hier dient somit die Einfüllkammer-Seitenwand in einer vorteilhaften Doppelfunktion gleichzeitig zur Anbindung der Gehäuseeinsatz-Bodenwand, so dass hierzu keine weiteren Maßnahmen erforderlich sind.

Die Einfüllkammer-Wandanordnung kann zudem gemäß einer besonders bevorzugten Ausführungsform eine zweite Einfüllkammer-Seitenwand aufweisen, die bei geschlossenen Spenderdeckel entlang einer zugeordneten Gehäuse-Seitenwand, vorzugsweise entlang und in Anlage an einer zugeordneten Gehäuse-Seitenwand, verläuft und/oder dort angebunden ist. Die zweite Einfüllkammer-Seitenwand ist im Wesentlichen parallel und beabstandet zu der ersten Einfüllkammer-Seitenwand ausgerichtet, wobei sich die zweite Einfüllkammer-Seitenwand in Richtung zur Rückwand hin winklig, vorzugsweise in etwa mit einem Winkel von 90 Grad, an die Einfüllkammer-Vorderwand anschließt und sich bis zur Rückwand erstreckt, bevorzugt an die Rückwand angrenzt, höchst bevorzugt an der Rückwand anliegt. Mit einem derartigen Aufbau, bei dem die zweite Einfüllkammer-Seitenwand in einer flächigen Anlageverbindung an der zugeordneten Gehäuse-Seitenwand angebunden werden kann, wird eine wesentliche Versteifung und damit eine wesentliche Stabilitätserhöhung für das Gehäuse insgesamt erzielt. Zudem gelingt damit eine vorteilhafte stabile Anordnung des Gehäuseeinsatzes im Gehäuseinnenraum.

In der zweiten Einfüllkammer-Seitenwand und der zugeordneten Gehäuse-Seitenwand kann jeweils ein im Wesentlichen gleicher und in Hochachsenrichtung verlaufender Sichtschlitz ausgebildet werden, mit dem der Füllstand in der Tampon-Aufnahmekammer sichtbar ist. Alternativ oder zusätzlich kann weiter vorgesehen sein, dass in der, der Slipeinlagen-Aufnahmekammer zugeordneten Gehäuse-Seitenwand ein in Hochachsenrichtung verlaufender Sichtschlitz ausgebildet ist, mit dem der Füllstand in der Slipeinlagen-Aufnahmekammer sichtbar ist. Mittels derartiger Sichtschlitze kann dann einfach von außen, ohne Öffnen des Spenderdeckels, ersehen werden, ob ein Nachfüllen der Slipeinlagen bzw. der Tampons eventuell erforderlich ist.

Die Tampon-Entnahmeöffnung weist bevorzugt eine kuppelförmige Geometrie mit einer in Querrichtung verlaufenden Unterkante, zwei sich von den Unterkantenenden weg nach oben, vorzugsweise geradlinig nach oben, erstreckenden und einander gegenüberliegenden Seitenkanten und einer kuppelfömig gewölbten Oberkante auf. Eine derartige Tampon-Entnahmeöffnung ist für ein ergonomischen Eingriff einer menschlichen Hand zur Tampon-Entnahme sehr gut geeignet. Die Tampon-Entnahme wird hierdurch wesentlich vereinfacht und erleichtert.

Die Slipeinlagen-Entnahmeöffnung ist bevorzugt durch einen sich in Querrichtung an der Unterkante der Vorderwand erstreckenden Längsschlitz gebildet, wobei an einer, bezogen auf die Hochachsenrichtung, Oberkante des Längsschlitzes, vorzugsweise in etwa mittig mit Bezug auf die Quererstreckung des Längsschlitzes und/oder in etwa auf gleicher Höhe wie die als Fingereingriffsöffnung fungierende Ausnehmung in der Gehäuseeinsatz-Bodenwand, eine Ausnehmung, vorzugsweise eine halbkreisförmige Ausnehmung, als Fingereingriffsöffnung vorgesehen ist. Dadurch kann insbesondere die unterste Slipeinlage einfach und bequem sowie ergonomisch aus der Slipeinlagen-Entnahmeöffnung entnommen werden.

Die Spendereinrichtung und ihre Bauteile kann aus jedem geeigneten Material hergestellt sein, so zum Beispiel aus einem Kunststoffmaterial oder aus einem Metall, wobei selbstverständlich auch Mischformen möglich sind. Beispielsweise kann das Gehäuse aus einem Metall, zum Beispiel aus einem Edelstahl hergestellt sein, während der Gehäuseeinsatz aus einem Kunststoffmaterial hergestellt ist, um nur ein mögliches Beispiel zu nennen.

Die Erfindung wurde vorstehend in Verbindung mit der bevorzugten Ausrichtung in Hochachsenrichtung beschrieben. Es versteht sich, dass diese Ausrichtung in Richtung der Hochachse lediglich beispielhaft zu verstehen ist und selbstverständlich auch davon abweichende Ausrichtungen mit der erfindungsgemäßen Spendereinrichtung grundsätzlich möglich sind, ohne das Erfindungsprinzip zu verlassen.

Die Erfindung wird nachstehend anhand einer Zeichnung näher erläutert.

Es zeigen:
- Figur 1: eine schematische, perspektivische Vorderansicht einer beispielhaften Ausführungsform einer erfindungsgemäßen Spendereinrichtung im geschlossenen Zustand,
- Figur 2: die Spendereinrichtung gemäß Figur 1 beim Öffnen des Spenderdeckels,
- Figur 3: eine perspektivische, auseinandergezogene Darstellung der Spendereinrichtung gemäß Figur 1 und Figur 2,
- Figur 4: eine schematische Seitenansicht der Spendereinrichtung der Figuren 1 bis 3,
- Figur 5: eine Rückansicht der Spendereinrichtung gemäß der Figuren 1 bis 4.

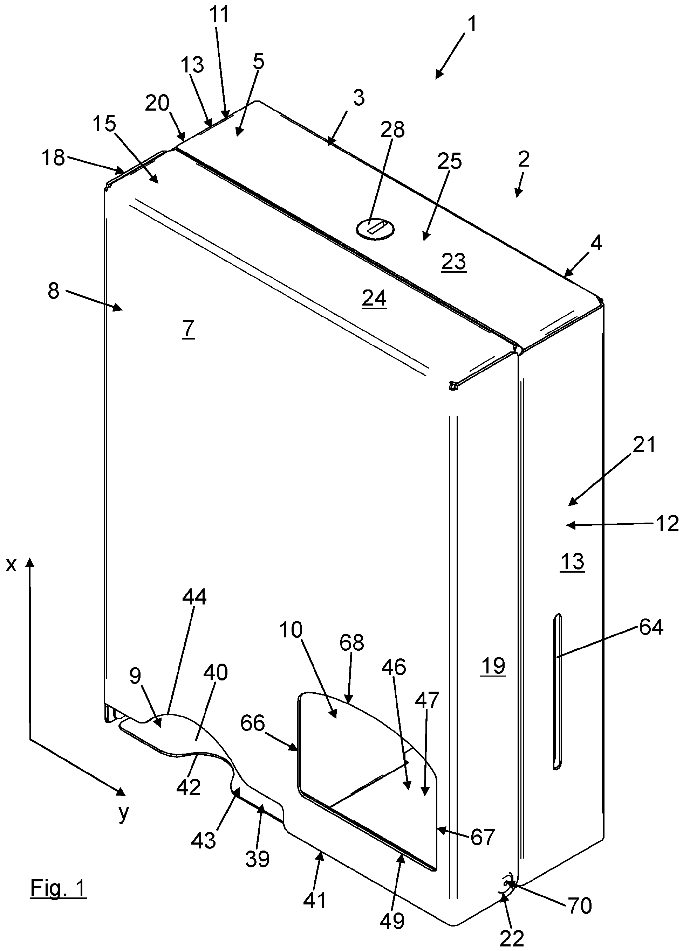

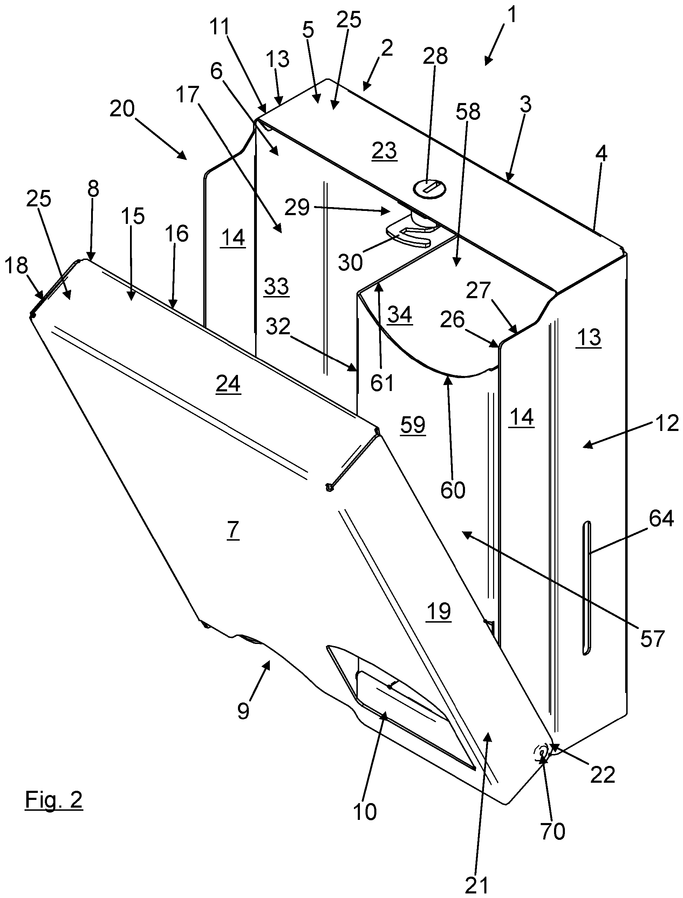

In der Figur 1 ist schematisch und beispielhaft eine perspektivische Darstellung einer beispielhaften Ausführungsform einer erfindungsgemäßen Spendereinrichtung 1 für Slipeinlagen und Tampons gezeigt, die ein rechteck- und kastenförmiges Gehäuse 2 aufweist. Wie dies insbesondere aus den Figuren 2, 3, 4 und 5 ersichtlich ist, weist das Gehäuse 2 einen Spenderkorpus 3 mit einer Rückwand 4 und einer sich daran nach vorne, von der Rückwand 4 erstreckenden Korpus-Wandanordnung 5 auf.

Der Spenderkorpus 3 weist weiter einen sich an die Rückwand 4 nach vorne hin anschließenden Aufnahmeraum 6 auf, der von der Korpus-Wandanordnung 5 zumindest teilweise umschlossen ist, wobei der spenderkorpusseitige Aufnahmeraum nach vorne hin, in Richtung von der Rückwand 4 weg, offen ist und mittels eines, eine Vorderwand 7 des Gehäuses 2 ausbildenden Spenderdeckels als einem weiteren Gehäuseteil lösbar verschließbar ist.

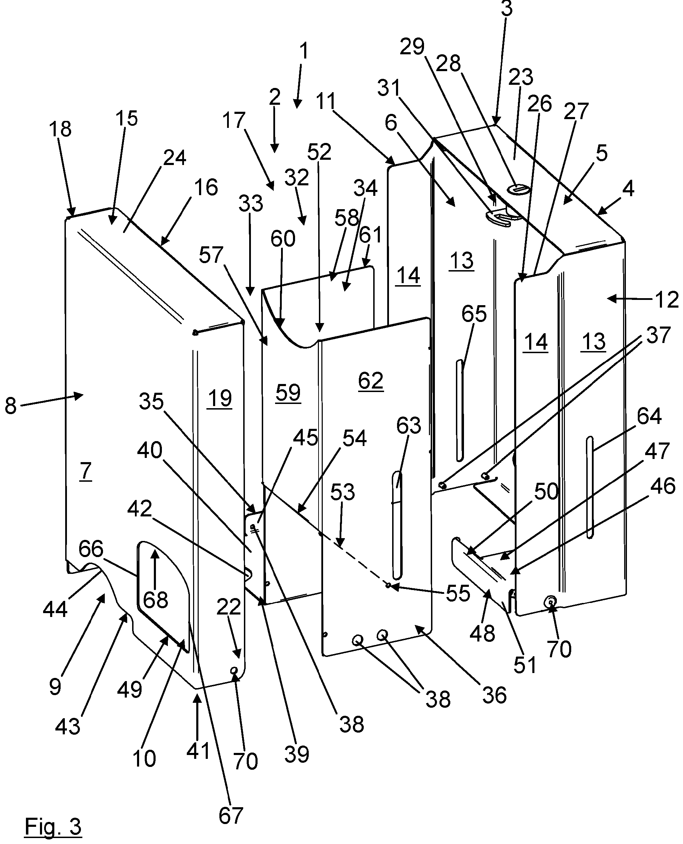

Wie dies insbesondere aus der Figur 1 und der Figur 3 ersichtlich ist, ist in der Vorderwand 7 eine Slipeinlagen-Entnahmeöffnung 9 und eine Tampon-Entnahmeöffnung 10 ausgebildet.

Der Spenderdeckel 8 ist hier am Spenderkorpus 3 schwenkbar angelenkt, und zwar in einem bezogen auf die Hochachsenrichtung x unteren Gehäusebereich. Wie dies insbesondere auch aus der Figur 2 ersichtlich ist, weist die Korpus-Wandanordnung 5 hierzu zwei, auf gegenüberliegenden Seiten des Aufnahmeraums 6 liegende und sich in Hochachsenrichtung x erstreckende Korpus-Seitenwände 11, 12 auf, an denen der Spenderdeckel 8 schwenkbar angelenkt ist (Schwenklager 70), so dass der Spenderdeckel 8, wie in der Figur 2 gezeigt, nach unten und vom Spenderkorpus 3 weg aufgeschwenkt werden kann.

Wie dies insbesondere sehr gut aus den Figuren 2 und 3 ersichtlich ist, sind die zwei Korpus-Seitenwände 11, 12 in einem, dem Spenderdeckel zugewandten vorderen freien Endbereich nach innen versetzt, insbesondere abgekröpft bzw. verjüngt, so dass die Korpus-Seitenwände 11, 12 jeweils einen hinteren Korpus-Seitenwandabschnitt 13 und einen sich daran nach vorne in Richtung zum Spenderdeckel 8 hin anschließenden, gegenüber dem hinteren Korpus-Seitenwandabschnitt 13 nach innen, in Richtung Aufnahmeraum 6 versetzten, vorderen Korpus-Seitenwandabschnitt 14 aufweisen.

Wie dies den Figuren weiter entnommen werden kann, schließt sich, bezogen auf den geschlossenen Zustand des Spenderdeckels 8, an den die Vorderwand 7 ausbildenden Bereich des Spenderdeckels 8 nach hinten, in Richtung zur Rückwand 4 bzw. zum Spenderkorpus 3 hin, eine Deckel-Wandanordnung 15 an, wobei der Spenderdeckel 8 weiter einen sich an die Vorderwand 7 nach hinten in Richtung in Rückwand 4 anschließenden deckelseitigen Aufnahmeraum 16 aufweist, der von der Deckel-Wandanordnung 15, hier beispielhaft lediglich bereichsweise, umschlossen ist und der zusammen mit dem korpusseitigen Aufnahmeraum 6 einen Gehäuseinnenraum 17 ausbildet.

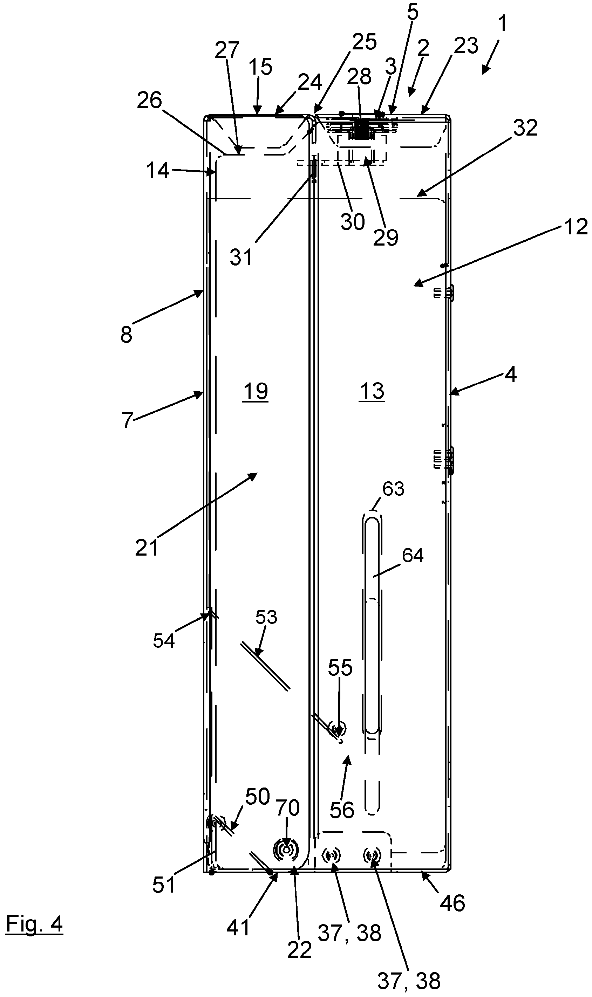

Wie dies insbesondere aus den Figuren 2 und 3 weiter ersichtlich ist, weist die Deckel-Wandanordnung 15 zwei dem Korpus-Seitenwänden 11, 12 zugeordnete Deckel-Seitenwände 18, 19 auf, die, bezogen auf den geschlossenen Zustand des Spenderdeckels 8 (siehe auch Figur 1 und die strichliert eingezeichneten Bereiche in der Figur 4), den nach innen versetzen vorderen Korpus-Seitenwandabschnitt 14 von außen her, vorzugsweise mit einem geringfügigen Spaltabstand umgreifen und im Wesentlichen in einer Flucht mit den jeweils zugeordneten hinteren Korpus-Seitenwandabschnitten 13 ausgerichtet sind, so dass die Deckel-Seitenwände 18, 19 und die jeweils zugeordneten hinteren Korpus-Seitenwandabschnitte 13 im geschlossenen Zustand des Spenderdeckels 8 und damit des Gehäuses 2 im Wesentlichen unmittelbar aneinander angrenzen und eine nach außen sichtbare gemeinsame Gehäuse-Seitenwand 20, 21 ausbilden.

Wie dies weitere insbesondere aus der Zusammenschau der Figuren 2, 3 und 4 ersichtlich ist, ist der Spenderdeckel 8 mit seinen Deckel-Seitenwänden 18, 19 schwenkbar im Bereich der vorderen Korpus-Seitenwandabschnitte 14 angelenkt, wobei die Anlenkung so durchgeführt ist bzw. die Deckel-Seitenwände 18, 19 so ausgebildet sind, dass der Spenderdeckel im aufgeschwenkten und damit geöffneten Zustand (siehe Figur 2) mit seinem schwenkachsenseitigen, unteren Deckel-Wandkantenbereich 22 von der Rückwand 4 beabstandet ist.

Die Korpus-Wandanordnung 5 weist weiter, wie dies insbesondere aus den Figuren 1 bis 4 ersichtlich ist, eine, bezogen auf einen in Hochachsenrichtung x oberen Bereich des Gehäuses 2, sich zwischen den hinteren Korpus-Seitenwandabschnitten 13 erstreckende Korpus-Oberwand 23 auf, an die im geschlossenen Zustand des Spenderdeckels eine, bezogen auf einen in Hochachsenrichtung x oberen Bereich des Gehäuses, Deckel-Oberwand 24, die sich zwischen den Deckel-Seitenwänden 18, 19 erstreckt, im Wesentlichen unmittelbar angrenzt bzw. anliegt. Wie aus den Figuren weiter ersichtlich ist, liegen die Deckel-Oberwand 24 und die Korpus-Oberwand 23 im geschlossenen Zustand des Spenderdeckels 8 im Wesentlichen in einer Flucht und bilden damit eine gemeinsame Gehäuse-Oberwand 25 aus.

Wie dies insbesondere aus der Zusammenschau der Figuren 2, 3 und 4 ersichtlich ist, ist ein vorderer Endkantenbereich 26 einer Oberkante 27 der vorderen Korpus-Seitenwandabschnitte 14 angefast bzw. angeschrägt und liegt tiefer als die Deckel-Oberwand 24, so dass beim Schließen des Spenderdeckels 8 weder im Bereich der vorderen Korpus-Seitenwandabschnitte 14 noch im Bereich des vorderen Endkantenbereichs 26 eine Behinderung des Schließvorgangs stattfindet. Vielmehr fädeln sich dann die vorderen Korpus-Seitenwandabschnitte 14 einfach hinter die Deckel-Wandanordnung 15, so dass der Schließvorgang geführt und ohne Verkanten oder dergleichen ablaufen kann.

Wie dies aus den Figuren 2, 3 und 4 weiter ersichtlich ist, liegt ein Betätigungselement 28 einer Schließvorrichtung 29 im Wesentlichen oberflächenbündig und von außen her zugänglich in der Korpus-Oberwand 23 ein, wobei das Betätigungselement 28 hier als Drehbetätigungselement ausgebildet ist, bei dessen Drehbetätigung ein mit dem Betätigungselement 28 wirkverbundenes Schließelement 30 der Schließvorrichtung, das hier beispielhaft durch einen Haken symbolisiert ist, in Eingriff und außer Eingriff mit einem im Gehäuseinnenraum 17 an der Deckel-Oberwand 24 angeordnetem Schließgegenelement 31 bringbar ist, das lediglich äußerst schematisch und strichliert in der Figur 4 gezeigt ist.

Wie dies weiter insbesondere aus der Zusammenschau der Figuren 2 bis 4 ersichtlich ist, ist im Gehäuseinnenraum 20 ein, hier bevorzugt und beispielhaft durch ein separates Bauteil gebildeter, Gehäuseeinsatz 32 angeordnet, der eine sich in Hochachsenrichtung x erstreckende Slipeinlagen-Aufnahmekammer 33 und eine davon getrennte, sich ebenfalls in Hochachsenrichtung x erstreckende Tampon-Aufnahmekammer 34 ausbildet. Die Tampon-Aufnahmekammer 34 mündet in der Tampon-Entnahmeöffnung 10, während die Slipeinlagen-Aufnahmekammer 33 in der Slipeinlagen-Entnahmeöffnung 9 mündet, was nachstehend noch näher beschrieben wird.

Aus den Figuren ist gut ersichtlich, dass die Tampon-Aufnahmekammer 34 und die Slipeinlagen-Aufnahmekammer 33 in Querrichtung y gesehen nebeneinander liegend angeordnet sind. Ebenso sind die Tampon-Entnahmeöffnung 10 und die Slipeinlagen-Entnahmeöffnung 9 in Querrichtung y gesehen nebeneinander in einem, bezogen auf die Hochachsenrichtung x, unteren Bereich der Vorderwand 7 angeordnet.

Im hier gezeigten Beispielfall erstreckt sich der Gehäuseeinsatz 32 im montierten Zustand zwischen der Rückwand 4 und der Vorderwand 7, und zwar dergestalt, dass der Gehäuseeinsatz 32 im Wesentlichen unmittelbar an die Vorderwand 7 bzw. an die Rückwand 4 angrenzt, wenn der Spenderdeckel 8 geschlossen ist (siehe insbesondere Figur 4).

Wie dies lediglich äußerst schematisch aus der Figur 3 ersichtlich ist, ist der Gehäuseeinsatz 32 mit in Querrichtung y gegenüberliegenden Gehäuseeinsatz-Seitenbereichen 35, 36 an den Korpus-Seitenwänden 11, 12 der Korpus-Wandanordnung 5 gehaltert. Die Halterung erfolgt hier lediglich beispielhaft über eine Nietverbindung, bei der, ebenfalls wiederum nur beispielhaft, Nietstifte 37, die an der Innenseite der Korpus-Seitenwände 11, 12 angeordnet sind, in Nietausnehmungen 38 der Gehäuseeinsatz-Seitenbereiche 35, 36 eingreifen und dann für eine feste Verbindung entsprechend umgenietet werden. Alternativ hierzu kann selbstverständlich auch eine Form- und/oder Kraftschlussverbindung vorgesehen sein, zum Beispiel eine Schraubverbindung, um nur ein Beispiel zu nennen.

Die Spendereinrichtung und ihre Bauteile kann aus jedem geeigneten Material hergestellt sein, so zum Beispiel aus einem Kunststoffmaterial oder aus einem Metall, wobei selbstverständlich auch Mischformen möglich sind. Beispielsweise kann das Gehäuse aus einem Metall, zum Beispiel aus einem Edelstahl hergestellt sein, während der Gehäuseeinsatz aus einem Kunststoffmaterial hergestellt ist, um nur ein mögliches Beispiel zu nennen.

Wie dies insbesondere aus den Figuren 1 und 3 weiter ersichtlich ist, weist der Gehäuseeinsatz 32 in einem, bezogen auf die Hochachsenrichtung x, unteren Bereich der Slipeinlagen-Aufnahmekammer 33 eine im Wesentlichen horizontal ausgerichtete und/oder plattenförmige Gehäuseeinsatz-Bodenwand 39 auf, die eine Auflagefläche 40 für eine unterste Slipeinlage (nicht dargestellt) eines ebenfalls nicht dargestellten Stapels aus mehreren, in der Slipeinlagen-Aufnahmekammer 33 aufgenommenen Slipeinlagen ausbildet.

Im hier gezeigten Ausführungsbeispiel ist vorgesehen, dass die Slipeinlagen-Aufnahmekammer 33 bei geschlossenem Spenderdeckel 8 von der Rückwand 4, der Vorderwand 7, der Gehäuseseitenwand 20 und einer nachstehend noch näher beschriebenen Wand der Tampon-Aufnahmekammer 34, nämlich der zweiten Einfüllkammer-Seitenwand 62, begrenzt ist und bei geöffnetem Deckel (Figur 2) zumindest nicht mehr durch die Vorderwand 7 begrenzt ist und damit für eine Beschickung mit Slipeinlagen von der Seite des Spenderdeckels 8 her und damit von vorne her frei zugänglich ist.

Die Gehäuseeinsatz-Bodenwand 39 begrenzt die Slipeinlagen-Entnahmeöffnung 9, die als unterkantenseitige Aussparung in der Vorderwand 7 ausgebildet ist, nach unten hin, wobei die Gehäuseeinsatz-Bodenwand 39 gleichzeitig auch das Gehäuse 2 nach unten hin begrenzt und damit einen Teil einer Gehäuse-Bodenwand 41 des Gehäuses ausbildet, wobei sich die Gehäuseeinsatz-Bodenwand 39 dergestalt zwischen der Vorderwand 7 und der Rückwand 4 erstreckt, dass sie dort im Wesentlichen unmittelbar angrenzt.

Die Gehäuseeinsatz-Bodenwand 39 weist an ihren der Slipeinlagen-Entnahmeöffnung 9 zugewandten Vorderkantenbereich, vorzugsweise in etwa mittig mit Bezug auf die Quererstreckung der Slipeinlagen-Entnahmeöffnung 9 in der Vorderwand 7, eine hier beispielhaft halbkreisförmig ausgebildete Ausnehmung 42 als Fingereingriffsöffnung auf.

Wie dies aus den Figuren weiter ersichtlich ist, ist die Slipeinlagen-Entnahmeöffnung 9 durch einen sich in Querrichtung an der Unterkante der Vorderwand 7 erstreckenden Längsschlitz 43 gebildet, wobei an einer, bezogen auf die Hochachsenrichtung x, Oberkante des Längsschlitzes 43, hier beispielhaft in etwa mittig mit Bezug auf die Querstreckung des Längsschlitzes 43 bzw. in etwa auf gleicher Höhe wie die als Fingereingriffsöffnung fungierende Ausnehmung 42 in der Gehäuseeinsatz-Bodenwand 39, eine hier beispielhaft halbkreisförmig ausgebildete Ausnehmung 44 als weitere Fingereingriffsöffnung vorgesehen ist. Dadurch können die Slipeinlagen bequem aus der Slipeinlagen-Entnahmeöffnung 9 entnommen werden.

Es versteht sich, dass die Slipeinlagen-Entnahmeöffnung 9 bzw. ihr Längsschlitz 43 so dimensioniert ist, dass jeweils immer nur eine unterste Slipeinlage aus der Slipeinlagen-Entnahmeöffnung 9 entnommen bzw. herausgezogen werden kann und dass nach dem Herausziehen einer untersten Slipeinlage dann die nächsthöhere Slipeinlage nach unten fällt und auf der Auflagefläche 40 entnahmebereit aufsitzt bzw. aufliegt.

Wie dies insbesondere aus der Figur 3 ersichtlich ist, dort aber lediglich äußerst schematisch dargestellt ist, grenzt die Gehäuseeinsatz-Bodenwand 41 mit einem, den Gehäuse-Seitenbereich 35 ausbildenden Verbindungsbereich an die Korpus-Seitenwand 11 an und ist dort mit der Korpus-Seitenwand in der zuvor beschriebenen beispielhaften Art und Weise verbunden. Dieser den Gehäuseeinsatz-Seitenbereich 35 ausbildende Verbindungsbereich ist hier weiter beispielhaft durch eine in Hochachsenrichtung x nach oben gerichtete Umbiegung 45 der Gehäuseeinsatz-Bodenwand 39 gebildet, die im montierten Zustand flächig an der zugeordneten Korpus-Seitenwand 11 anliegt.

Wie dies weiter insbesondere aus der Figur 3 und mit Abstrichen aus Figur 4 ersichtlich ist, weist die Korpus-Wandanordnung 5, bezogen auf einen in Hochachsenrichtung x unteren Bereich des Gehäuses 2, eine Korpus-Bodenwand 46 auf, die sich hier beispielhaft lediglich im Bereich unterhalb der Tampon-Aufnahmekammer 34 erstreckt und somit im Bereich der Gehäuseeinsatz-Bodenwand 39 ausgespart ist. Diese Korpus-Bodenwand erstreckt sich im Bereich unterhalb der Tampon-Aufnahmekammer 34 zwischen der Vorderwand 7 und der Rückwand 4, wobei bevorzugt vorgesehen ist, dass die Korpus-Bodenwand 46 im Wesentlichen unmittelbar an die Vorderwand 7 und an die Rückwand 4 angrenzt.

Im montierten Zustand ist zudem bevorzugt vorgesehen, dass die Korpus-Bodenwand 46 unmittelbar an die Gehäuseeinsatz-Bodenwand 39 anschließt, und zwar bevorzugt dergestalt, dass die Gehäuseeinsatz-Bodenwand 39 und die Korpus-Bodenwand 46 im Wesentlichen in einer Flucht liegen und im geschlossenen Zustand des Spenderdeckels 8 und damit des Gehäuses 2 die gemeinsame Gehäuse-Bodenwand 41 ausbilden.

Die Korpus-Bodenwand 46 bildet weiter eine Auflagefläche 47 für Tampons aus und grenzt mit einem der Vorderwand 7 zugewandten Vorderkantenbereich 48 dergestalt an den Unterkantenbereich der in der Vorderwand 7 ausgebildeten Tampon-Entnahmeöffnung 10 an, dass die Korpus-Bodenwand 46 und damit die Auflagefläche 47, bezogen auf die Hochachsenrichtung x, tiefer liegt als eine Unterkante 49 der Tampon-Entnahmeöffnung.

Wie dies weiter aus der Figur 3 ersichtlich ist, weist der der Vorderwand 7 zugewandte Vorderkantenbereich 48 der Korpus-Bodenwand 46 eine, bezogen auf die Hochachsenrichtung x, konvexe und oberseitig abgerundete Aufwölbung 50 auf, von der ausgehend ein optionaler und nicht zwingend vorzusehender Anlageschenkel 51 im Wesentlichen geradlinig nach unten abragen kann, wobei dieser Anlageschenkel 51 bei geschlossenem Spenderdeckel 8 bevorzugt ohne Spaltabstand bzw. in einer flächigen Anlage an die Vorderwand 7 angrenzt. Wie dies insbesondere auch aus der Figur 4 ersichtlich ist, kann die Aufwölbung 50 hier in Richtung zur Auflagefläche 47 hin eine Schrägstellung aufweisen. Dadurch können die Tampons bei der Entnahme über die Tampon-Entnahmeöffnung 10 sozusagen mit den Fingern entlang der Aufwölbung 50 nach oben "herausgerollt" werden, was eine bequeme und komfortable Entnahme bedeutet.

Wie dies weiter insbesondere aus der Zusammenschau der Figuren 2 und 3 ersichtlich ist, weist die Tampon-Aufnahmekammer 34, bezogen auf die Hochachsenrichtung x, im Bereich oberhalb der Korpus-Bodenwand 46 eine Einfüllkammer 52 auf, deren Einfüllkammer-Bodenwand beabstandet oberhalb der Korpus-Bodenwand 46 liegt und als Schrägwand 53 ausgebildet ist, die von einem der Vorderwand 7 zugewandten ersten Schrägwandbereich 54 ausgehend in Richtung zur Rückwand 4 hin schräg abfällt, dergestalt, dass ein dem ersten Schrägwandbereich 54 gegenüberliegender, der Rückwand 4 zugewandter zweiter Schrägwandbereich 55, der durch dessen endseitige Schrägwandkante gebildet ist, einen Spaltabstand zur Rückwand 4 aufweist. Dieser Spaltabstand ist so bemessen, dass über ihn Tampons von der Einfüllkammer 52 in den Bereich der Korpus-Bodenwand 46 gelangen können. Hierzu ist der Spaltabstand im gezeigten Beispielfall durch einen sich in Querrichtung y erstreckenden Längsschlitz 56 gebildet, so dass die Tampons nur entsprechend quer ausgerichtet durch den Längsschlitz 56 nach unten auf die Korpus-Bodenwand 46 fallen können. Es versteht sich, dass der Längsschlitz 56 eine Größe aufweist, die um ein definiertes Maß größer ist als der größte in der Einfüllkammer 52 aufnehmbare Tampon. Die Einfüllkammer 52 ist im hier gezeigten Ausführungsbeispiel in Umfangsrichtung gesehen durch eine Einfüllkammer-Wandanordnung 57, die Rückwand 4 und die Korpus-Seitenwand 12 begrenzt und weist an ihrer, bezogen auf die Hochachsenrichtung x, Oberseite eine obere Einfüllöffnung 58 auf, die, wie dies aus der Figur 2 ersichtlich ist, im geöffneten Zustand des Spenderdeckels 8 für eine Befüllung mit Tampons zugänglich ist.

Die Einfüllkammer 52 erstreckt sich, bezogen auf die Hochachsenrichtung x, so weit nach oben, dass die Einfüllöffnung 58 mit einem Spaltabstand an die Korpus-Oberwand 23 angrenzt.

Die Einfüllkammer-Wandanordnung 57 weist eine bei geschlossenem Spenderdeckel der Vorderwand 7 zugeordnete Einfüllkammer-Vorderwand 59 auf, die bei geöffnetem Spenderdeckel 8 von außen sichtbar ist, wobei die Einfüllkammer-Vorderwand 59 bei geschlossenem Spenderdeckel 8 im Wesentlichen parallel zur Vorderwand 7 ausgerichtet ist und bevorzugt, wie lediglich beispielhaft in der Figur 4 gezeigt, unmittelbar an die Vorderwand 7 angrenzt, insbesondere dort flächig anliegt.

Die Einfüllkammer-Vorderwand 59 weist zudem hier beispielhaft im Bereich ihrer, bezogen auf die Hochachsenrichtung x, Oberkante eine hier lediglich beispielhaft halbkreisförmig ausgebildete Ausnehmung 60 auf, die die obere Einfüllöffnung 58 als seitliche Einfüllöffnung erweitert, und zwar insbesondere für den in der Figur 2 dargestellten Fall, dass die obere Einfüllöffnung 58 bei geöffnetem Spenderdeckel 8 teilweise von einer Korpus-Oberwand 23 überdeckt ist.

Die Einfüllkammer-Wandanordnung 57 weist weiter eine erste Einfüllkammer-Seitenwand 61 auf, die sich zur Abtrennung der Tampon-Aufnahmekammer 34 von der Slipeinlagen-Aufnahmekammer 33 in einem hier in etwa mittleren Bereich des Gehäuseinnenraums 17 in Richtung zur Rückwand hin, hier beispielhaft in einem Winkel von 90 Grad, an die Einfüllkammer-Vorderwand 59 anschließt und sich bis zur Rückwand 4 erstreckt, an der sie bevorzugt anliegt.

Wie insbesondere aus der Figur 3 gut ersichtlich ist, ist die Gehäuseeinsatz-Bodenwand 39 in einem bezogen auf die Hochachsenrichtung x unteren Bereich der ersten Einfüllkammer-Seitenwand 61 an der Einfüllkammer-Seitenwand 61 angebunden und ragt dort von dieser im Wesentlichen horizontal ab.

Die Einfüllkammer-Wandanordnung 57 weist weiter eine zweite Einfüllkammer-Seitenwand 62 auf, die bei geschlossenem Spenderdeckel entlang und in Anlage an der zugeordneten Gehäuse-Seitenwand 21 verläuft und dort, wie bereits zuvor erläutert, angebunden ist. Insofern bildet die zweite Einfüllkammer-Seitenwand 62 hier gleichzeitig auch den Gehäuseeinsatz-Seitenbereich 36 aus.

Die zweite Einfüllkammer-Seitenwand 62 verläuft dabei im Wesentlichen parallel und beabstandet zu der ersten Einfüllkammer-Seitenwand 61, wobei sich die zweite Einfüllkammer-Seitenwand 62 hier in Richtung zur Rückwand 4 hin unter einem Winkel von in etwa 90 Grad an die Einfüllkammer-Vorderwand 59 anschließt und sich ebenfalls wiederum bevorzugt bis zur Rückwand 4 erstreckt, bevorzugt an die Rückwand angrenzt bzw. dort anliegt.

Die zweite Einfüllkammer-Seitenwand 62 weist hier, wie dies in der Figur 3 lediglich beispielhaft dargestellt ist, bevorzugt eine gleiche Länge, jeweils in Hochachsenrichtung x gesehen, auf wie die erste Einfüllkammer-Seitenwand 61, erstreckt sich also im Wesentlichen über die gesamte Höhe des Gehäuseinnenraums 17.

In der zweiten Einfüllkammer-Seitenwand 62 und der zugeordneten Gehäuse-Seitenwand 21 ist jeweils ein im Wesentlichen gleicher und in Hochachsenrichtung x verlaufender Sichtschlitz 63, 64 ausgebildet, mit dem der Füllstand in der Tampon-Aufnahmekammer 34 sichtbar ist.

Analog dazu ist in der, der Slipeinlagen-Aufnahmekammer 33 zugeordneten Gehäuse-Seitenwand 20 ein in Hochachsenrichtung x verlaufender Sichtschlitz 65 ausgebildet, mit dem der Füllstand in der Slipeinlagen-Aufnahmekammer 33 sichtbar ist.

Wie dies weiter insbesondere aus der Figur 1 und der Figur 3 ersichtlich ist, weist die Tampon-Entnahmeöffnung 10 eine kuppelförmige Geometrie mit der Unterkante 49, zwei sich von den Enden der Unterkante 49 weg nach oben, insbesondere geradlinig nach oben, erstreckenden und einander gegenüberliegenden Seitenkanten 66, 67 und einer kuppelförmig gewölbten Oberkante 68 auf.

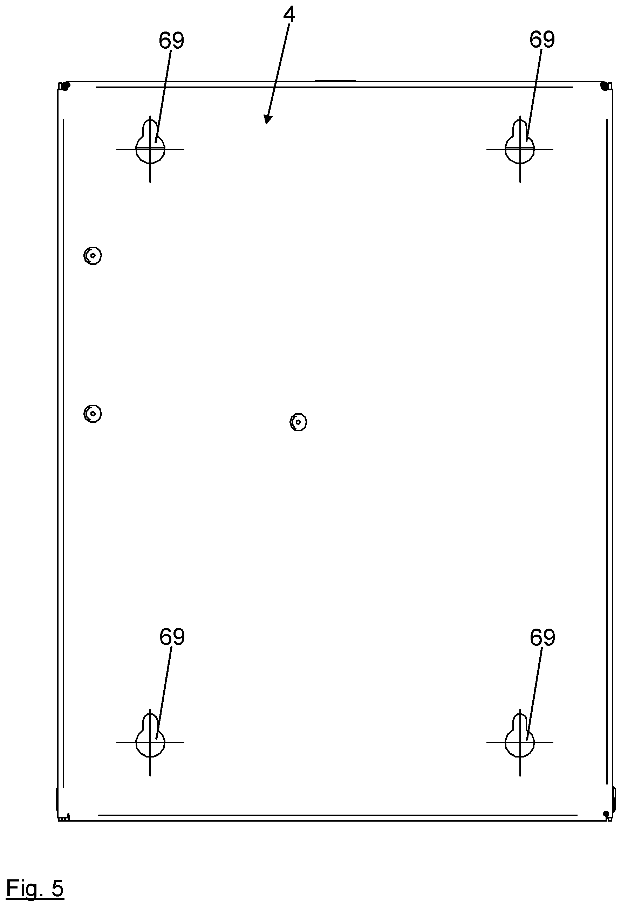

In der Figur 5 ist schließlich eine Rückansicht der Rückwand 4 dargestellt, die mehrere Öffnungen 69 aufweist, mittels denen die Rückwand 4 beispielsweise an einer Gebäudewand mittels einer Schraubverbinding befestigt werden kann (nicht dargestellt).

**Bezugszeichenliste**

| | | | |
|---|---|---|---|
| 1 | Spendereinrichtung | 31 | Schließgegenelement |
| 2 | Gehäuse | 32 | Gehäuseeinsatz |
| 3 | Spenderkorpus | 33 | Slipeinlagen-Aufnahmekammer |
| 4 | Rückwand | 34 | Tampon-Aufnahmekammer |
| 5 | Korpus-Wandanordnung | 35 | Gehäuseeinsatz-Seitenbereich |
| 6 | Aufnahmeraum | 36 | Gehäuseeinsatz-Seitenbereich |
| 7 | Vorderwand | 37 | Nietstifte |
| 8 | Spenderdeckel | 38 | Nietausnehmungen |
| 9 | Slipeinlagen-Entnahmeöffnung | 39 | Gehäuseeinsatz-Bodenwand |
| 10 | Tampon-Entnahmeöffnung | 40 | Auflagefläche |
| 11 | Korpus-Seitenwand | 41 | Gehäuse-Bodenwand |
| 12 | Korpus-Seitenwand | 42 | Ausnehmung |
| 13 | hinterer Korpus-Seitenwandabschnitt | 43 | Längsschlitz |
| 14 | vorderer Korpus-Seitenwandabschnitt | 44 | Ausnehmung |
| 15 | Deckel-Wandanordnung | 45 | Umbiegung |
| 16 | Aufnahmeraum | 46 | Korpus-Bodenwand |
| 17 | Gehäuseinnenraum | 47 | Auflagefläche |
| 18 | Deckel-Seitenwand | 48 | Vorderkantenbereich |
| 19 | Deckel-Seitenwand | 49 | Unterkante |
| 20 | Gehäuse-Seitenwand | 50 | Aufwölbung |
| 21 | Gehäuse-Seitenwand | 51 | Anlageschenkel |
| 22 | unterer Deckel-Wandkantenbereich | 52 | Einfüllkammer |
| 23 | Korpus-Oberwand | 53 | Schrägwand |
| 24 | Deckel-Oberwand | 54 | erster Schrägwandbereich |
| 25 | Gehäuse-Oberwand | 55 | zweiter Schrägwandbereich |
| 26 | vorderer Endkantenbereich | 56 | Längsschlitz |
| 27 | Oberkante | 57 | Einfüllkammer-Wandanordnung |
| 28 | Betätigungselement | 58 | obere Einfüllöffnung |
| 29 | Schließvorrichtung | 59 | Einfüllkammer-Vorderwand |
| 30 | Schließelement | 60 | Ausnehmung |
| 61 | erste Einfüllkammer-Seitenwand | | |
| 62 | zweite Einfüllkammer-Seitenwand | | |
| 63 | Sichtschlitz | | |
| 64 | Sichtschlitz | | |
| 65 | Sichtschlitz | | |
| 66 | Seitenkante | | |
| 67 | Seitenkante | | |
| 68 | Oberkante | | |
| 69 | Öffnungen | | |
| 70 | Schwenklager | | |

## Patentansprüche

1. Spendereinrichtung für Slipeinlagen und Tampons,
**gekennzeichnet durch**
ein Gehäuse (2), das ausgebildet ist, in dem Gehäuseinnenraum (17) mehrere Slipeinlagen und mehrere Tampons getrennt voneinander aufzunehmen,
wobei das Gehäuse (2) eine Slipeinlagen-Entnahmeöffnung (9), über die die Slipeinlagen entnehmbar sind, und eine Tampon-Entnahmeöffnung (10), über die die Tampons entnehmbar sind, aufweist,
wobei das Gehäuse (2) kastenförmig, insbesondere rechteck- und kastenförmig, ausgebildet ist und einen Spenderkorpus (3) mit einer Rückwand (4) und einer sich nach vorne, von der Rückwand (4) weg erstreckenden Korpus-Wandanordnung (5) aufweist,
wobei der Spenderkorpus (3) einen sich an die Rückwand (4) nach vorne hin anschließenden Aufnahmeraum (6) aufweist, der wenigstens einen Bestandteil des Gehäuseinnenraums (17) ausbildet und der von der Korpus-Wandanordnung (5) wenigstens bereichsweise umschlossen ist, wobei der spenderkorpusseitige Aufnahmeraum (6) nach vorne hin, in Richtung von der Rückwand (4) weg, offen ist und mittels eines, eine Vorderwand (7) des Gehäuses (2) ausbildenden Spenderdeckels (8) als weiterem Gehäuseteil lösbar verschließbar ist,
wobei die Slipeinlagen-Entnahmeöffnung (9) und die Tampon-Entnahmeöffnung (10) in der Vorderwand (7) ausgebildet sind.

2. Spendereinrichtung nach Anspruch 1, **dadurch gekennzeichnet, dass** der Spenderdeckel (8) am Spenderkorpus (3) schwenkbar angelenkt ist, höchst bevorzugt in einem, bezogen auf die Hochachsenrichtung, unteren Endbereich des Spenderkorpus (3) schwenkbar angelenkt ist.

3. Spendereinrichtung nach Anspruch 1 oder 2, **dadurch gekennzeichnet, dass** die Korpus-Wandanordnung (5) zwei auf gegenüberliegenden Seiten des Aufnahmeraums (6) liegende und sich in Hochachsenrichtung erstreckende Korpus-Seitenwände (11, 12) aufweist, an denen der Spenderdeckel (8) schwenkbar angelenkt ist, wobei bevorzugt vorgesehen ist, dass der Spenderdeckel (8) jeweils an einem bezogen auf die Hochachsenrichtung unteren Endbereich der Korpus-Seitenwände (11, 12) schwenkbar angelenkt ist, so dass der Spenderdeckel (8) nach unten aufschwenkbar ist.

4. Spendereinrichtung nach einem der vorhergehenden Ansprüche, **dadurch gekennzeichnet, dass** sich, bezogen auf den geschlossenen Zustand des Spenderdeckels (8), an den die Vorderwand (7) ausbildenden Bereich des Spenderdeckels (8) nach hinten, in Richtung zur Rückwand (4) hin, eine Deckel-Wandanordnung (15) anschließt, wobei bevorzugt vorgesehen ist, dass der Spenderdeckel (8) einen sich an die Vorderwand (7) nach hinten in Richtung Rückwand (4) anschließenden deckelseitigen Aufnahmeraum (16) aufweist, der von der Deckel-Wandanordnung (15) wenigstens bereichsweise umschlossen ist und der zusammen mit dem korpusseitigen Aufnahmeraum (6) den Gehäuseinnenraum (17) ausbildet.

5. Spendereinrichtung nach Anspruch 3 und 4, **dadurch gekennzeichnet,**
**dass** die Deckel-Wandanordnung (15) zwei den Korpus-Seitenwänden (11, 12) zugeordnete Deckel-Seitenwände (18, 19) aufweist,
**dass** die zwei Korpus-Seitenwände (11, 12) in einem, dem Spenderdeckel (8) zugewandten vorderen freien Endbereich nach innen versetzt, insbesondere abgekröpft und/oder verjüngt, sind, so dass die Korpus-Seitenwände (11, 12) jeweils einen hinteren Korpus-Seitenwandabschnitt (13) und einen sich daran nach vorne in Richtung zum Spenderdeckel (8) hin anschließenden, gegenüber dem hinteren Korpus-Seitenwandabschnitt (13) nach innen, in Richtung Aufnahmeraum (6) versetzten, vorderen Korpus-Seitenwandabschnitt (14) aufweisen,
**dass** die Deckel-Seitenwände (18, 19), bezogen auf den geschlossenen Zustand des Spenderdeckels (8), den nach innen versetzten vorderen Korpus-Seitenwandabschnitt (14) von außen her umgreifen, vorzugsweise mit einem Spaltabstand umgreifen, und im Wesentlich in einer Flucht mit den jeweils zugeordneten hinteren Korpus-Seitenwandabschnitten (14) ausgerichtet sind, wobei bevorzugt vorgesehen ist, dass die Deckel-Seitenwände (18, 19) und die jeweils zugeordneten hinteren Korpus-Seitenwandabschnitte (13) im geschlossenen Zustand des Spenderdeckels (8) und damit des Gehäuses (2) im Wesentlichen unmittelbar aneinander angrenzen und eine nach außen sichtbare gemeinsame Gehäuse-Seitenwand (20, 21) ausbilden.

6. Spendereinrichtung nach Anspruch 5, **dadurch gekennzeichnet, dass** der Spenderdeckel (8) mit seinen Deckel-Seitenwänden (18, 19) schwenkbar im Bereich der vorderen Korpus-Seitenwandabschnitte (14) angelenkt ist, wobei bevorzugt vorgesehen ist, dass die Anlenkung so durchgeführt ist und/oder die Deckel-Seitenwände (18, 19) so ausgebildet sind, dass der Spenderdeckel (8) im aufgeschwenkten und damit geöffneten Zustand, insbesondere in einem um 90 Grad von dem Spenderkorpus (3) weg aufgeschwenkten und geöffneten Zustand, mit seinem schwenkachsenseitigen unteren Deckel-Wandkantenbereich (22) von der Rückwand (4) beabstandet ist.

7. Spendereinrichtung nach Anspruch 5 oder 6, **dadurch gekennzeichnet, dass** die Korpus-Wandanordnung (5) eine, bezogen auf einen in Hochachsenrichtung oberen Bereich des Gehäuses (2), sich wenigstens zwischen den hinteren Korpus-Seitenwandabschnitten (13) erstreckende Korpus-Oberwand (23) aufweist, an die im geschlossenen Zustand des Spenderdeckels (8) eine, bezogen auf einen in Hochachsenrichtung oberen Bereich des Gehäuses (2), sich zwischen den Deckel-Seitenwänden (18, 19) erstreckende Deckel-Oberwand (24) im Wesentlichen unmittelbar angrenzt und/oder anliegt, wobei bevorzugt vorgesehen ist, dass die Deckel-Oberwand (24) und die Korpus-Oberwand (23) im Wesentlichen in einer Flucht liegen und im geschlossenen Zustand des Spenderdeckels (8) und damit des Gehäuses eine gemeinsame Gehäuse-Oberwand (25) bilden.

8. Spendereinrichtung nach einem der vorhergehenden Ansprüche, **dadurch gekennzeichnet,**
**dass** im Gehäuseinnenraum (17) ein, vorzugsweise durch ein separates Bauteil gebildeter, Gehäuseeinsatz (32) angeordnet ist, der eine sich in Hochachsenrichtung erstreckende Slipeinlagen-Aufnahmekammer (33) und eine davon getrennte, sich ebenfalls in Hochachsenrichtung erstreckende Tampon-Aufnahmekammer (34) ausbildet,
**dass** die Tampon-Aufnahmekammer (34) in der Tampon-Entnahmeöffnung (10) und die Slipeinlagen-Aufnahmekammer (33) in der Slipeinlagen-Entnahmeöffnung (9) mündet.

9. Spendereinrichtung nach Anspruch 8, **dadurch gekennzeichnet, dass** der Gehäuseeinsatz (32) mittels einer Klemm- und/oder Schraub- und/oder Rastverbindung lösbar an dem Spenderkorpus (3), vorzugsweise wenigstens an den Korpus-Seitenwänden (11, 12) der Korpus-Wandanordnung (5), gehaltert ist, und/oder dass der Gehäuseeinsatz (32) mit dem Spenderkorpus (3), vorzugsweise wenigstens mit den Korpus-Seitenwänden (11, 12) der Korpus-Wandanordnung (5), durch wenigstens eine nur durch Zerstörung lösbare Verbindung verbunden ist, vorzugsweise mittels wenigstens einer Nietverbindung (37, 38) und/oder wenigstens einer Klebeverbindung.

10. Spendereinrichtung nach Anspruch 8 oder 9, **dadurch gekennzeichnet, dass** der Gehäuseeinsatz (32) in einem, bezogen auf die Hochachsenrichtung, unteren Bereich der Slipeinlagen-Aufnahmekammer (33) eine, vorzugsweise im Wesentlichen horizontal ausgerichtete und/oder plattenförmige, Gehäuseeinsatz-Bodenwand (39) aufweist, die eine Auflagefläche (40) für wenigstens eine darauf aufliegende und in der Slipeinlagen-Aufnahmekammer (33) aufgenommene Slipeinlage ausbildet, vorzugsweise eine Auflagefläche (40) für eine unterste Slipeinlage eines Stapels aus mehreren, in der Slipeinlagen-Aufnahmekammer (33) aufgenommenen Slipeinlagen ausbildet, wobei bevorzugt vorgesehen ist, dass die Slipeinlagen-Aufnahmekammer (33) bei geschlossenem Spenderdeckel (8) von der Rückwand (4), der Vorderwand (7), einer Gehäuse-Seitenwand (20) und einer Wand der Tampon-Aufnahmekammer (34) begrenzt ist und bei geöffnetem Spenderdeckel (8) zumindest nicht mehr durch die Vorderwand (7) begrenzt ist und damit für eine Beschickung mit Slipeinlagen frei zugänglich ist.

11. Spendereinrichtung nach einem der vorhergehenden Ansprüche, **dadurch gekennzeichnet, dass** die Korpus-Wandanordnung (5), bezogen auf einen in Hochachsenrichtung unteren Bereich des Gehäuses (2), eine Korpus-Bodenwand (46) aufweist, die sich wenigstens im Bereich unterhalb der Tampon-Aufnahmekammer (34) erstreckt, bevorzugt sich wenigstens im Bereich unterhalb der Tampon-Aufnahmekammer (34) zwischen der Vorderwand (7) und der Rückwand (4) erstreckt, höchst bevorzugt im Wesentlichen unmittelbar an die Vorderwand (7) und an die Rückwand (4) angrenzt.

12. Spendereinrichtung nach Anspruch 10 und 11, **dadurch gekennzeichnet, dass** die Korpus-Bodenwand (46) im Bereich der Gehäuseeinsatz-Bodenwand (39) ausgespart ist und sich unmittelbar an die Gehäuseeinsatz-Bodenwand (39) anschließt, wobei bevorzugt vorgesehen ist, dass die Gehäuseeinsatz-Bodenwand (39) und die Korpus-Bodenwand (46) im Wesentlichen in einer Flucht liegen und im geschlossenen Zustand des Spenderdeckels (8) und damit des Gehäuses (2) eine gemeinsame Gehäuse-Bodenwand (41) bilden.

13. Spendereinrichtung nach Anspruch 11 oder 12, **dadurch gekennzeichnet, dass** die Tampon-Aufnahmekammer (34), bezogen auf die Hochachsenrichtung, im Bereich oberhalb der Korpus-Bodenwand (46) eine Einfüllkammer (52) aufweist, deren Einfüllkammer-Bodenwand beabstandet oberhalb der Korpus-Bodenwand (46) liegt, dass die Einfüllkammer-Bodenwand als Schrägwand (53) ausgebildet ist, die von einem der Vorderwand (7) zugewandten ersten Schrägwandbereich (54) ausgehend in Richtung zur Rückwand (4) hin schräg abfällt, dergestalt, dass ein dem ersten Schrägwandbereich (54) gegenüberliegender, der Rückwand (4) zugewandter zweiter Schrägwandbereich, vorzugsweise dessen endseitige Schrägwandkante (55), einen Spaltabstand zur Rückwand (4) aufweist, wobei der Spaltabstand zur Rückwand (4) so bemessen ist, dass über ihn Tampons von der Einfüllkammer (52) in den Bereich der Korpus-Bodenwand (46) gelangen können.

14. Spendereinrichtung nach Anspruch 13, **dadurch gekennzeichnet, dass** die Einfüllkammer (52) in Umfangsrichtung gesehen wenigstens bereichsweise durch eine Einfüllkammer-Wandanordnung (57), bevorzugt durch eine Einfüllkammer-Wandanordnung (57) und die Rückwand (4), höchst bevorzugt durch eine Einfüllkammer-Wandanordnung (57), die Rückwand (4) und eine Korpus-Seitenwand (21), begrenzt ist und an ihrer, bezogen auf die Hochachsenrichtung, Oberseite eine obere Einfüllöffnung (58) aufweist, vorzugsweise die Einfüllkammer (52) oben offen ausgebildet ist, dass die obere Einfüllöffnung (52) im geöffneten Zustand des Spenderdeckels (8) für eine Befüllung mit Tampons zugänglich ist.

15. Spendereinrichtung nach Anspruch 14, **dadurch gekennzeichnet, dass** Einfüllkammer-Wandanordnung (57) eine bei geschlossenem Spenderdeckel (8) der Vorderwand (7) zugeordnete Einfüllkammer-Vorderwand (59) aufweist, die bei geöffnetem Spenderdeckel (8) von außen sichtbar ist, wobei bevorzugt vorgesehen ist, dass die Einfüllkammer-Vorderwand (59) bei geschlossenem Spenderdeckel (8) im Wesentlichen parallel zur Vorderwand (7) ausgerichtet ist und/oder an die Vorderwand (7) angrenzt, höchst bevorzugt flächig an der Vorderwand (7) anliegt.
